# EUROPEAN PATENT SPECIFICATION

(11) **EP 4 287 999 B1**
(45) Date of publication and mention of the grant of the patent: **03.06.2026**
(21) Application number: 22705968.0
(22) Date of filing: 07.02.2022
(51) Int. Cl.: A61F 5/44, A61M 1/00, A61F 5/451

(54) **PIVOTABLE FLUID COLLECTION DEVICE TUBING CONNECTORS AND RELATED SYSTEMS AND METHODS**
SCHWENKBARE SCHLAUCHVERBINDER FÜR FLÜSSIGKEITSSAMMELVORRICHTUNG SOWIE ZUGEHÖRIGE SYSTEME UND VERFAHREN
RACCORDS DE TUBE DE DISPOSITIF DE COLLECTE DE FLUIDE PIVOTANT ET SYSTÈMES ET PROCÉDÉS ASSOCIÉS

(30) Priority: 08.02.2021 US 202163147013 P
(43) Date of publication of application: 13.12.2023
(60) Divisional application: 26174447.8
(73) Proprietor: Purewick Corporation, El Cajon, California 92020 (US)
(72) Inventor: NEWTON, Camille Rose, Bonsall, California 92003 (US); DAVIS, Kathleen, Atlanta, Georgia 30307 (US)
(74) Representative: Hoffmann Eitle
(86) International application number: PCT/US2022/015471
(87) International publication number: WO 2022/170182

(56) References cited:
- US-A- 3 349 768
- US-A- 4 886 508
- US-A1- 2018 228 642

## Description

### CROSS-REFERENCE TO RELATED APPLICATIONS

This application claims priority to U.S. Provisional Patent Application No. 63/147,013 filed on February 8, 2021.

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters may be uncomfortable, painful, and may cause urinary tract infections. Conventional urine collection devices also may be limited when a patient is confined to a bed in a supine position. Tubing associated with urine collection devices is easily kinked or pinched, thereby inhibiting removal of urine from the urine collection devices. US 2018/228642 A1 discloses devices, systems, and methods for collecting urine discharged from the body of a user and carrying the urine away from the body. US 3349768 A discloses a suction evacuated urinal which is shaped to provide full tissue support for the external genitalia when positioned in contact therewith. US 4886508 A discloses a soft flexible memory retaining ladies catheter assembly and the preamble of claim 1.

Thus, users and manufacturers of fluid collection devices continue to seek new and improved devices, systems, and methods to collect urine.

### SUMMARY

Embodiments disclosed herein are related to pivotable and/or bendable tube connectors for fluid collection devices, systems, and methods. According to a first aspect, there is provided a fluid collection system according to claim 1.

According to a second aspect, there is provided a method of collecting fluid according to claim 10. The method includes positioning a fluid collection device on a user with a fluid permeable body of the fluid collection device at least proximate to a skin of the user. The fluid collection device includes a sump positioned within the fluid collection device and a fluid impermeable barrier. The method also includes adjusting a position of a first end region of a tube connector in fluid communication with the sump relative to a second end region of the tube connector whilst functionally maintaining the fluid communication between the second end region and the sump to orient the tube connector to a selected orientation. The method also includes drawing fluid discharged in the fluid collection device from the sump, through the tube connector, and into a fluid collection container.

Features from any of the disclosed embodiments may be used in combination with one another, without limitation as long as the resulting combination falls within the scope of the claims. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a block diagram of a fluid collection system, according to an embodiment.
**FIG. 2A** is a side view of a conventional urine collection system positioned on a user.
**FIG. 2B** is a side view of a female urine collection system having a tube connector, according to an embodiment.
**FIG. 2C** is a side view of the female urine collection system of **FIG. 2B** positioned on a user, according to an embodiment.
**FIG. 3A** is front view of a male urine collection system having a tube connector, according to an embodiment.
**FIG. 3B** is a front view of a male urine collection system having a tube connector, according to an embodiment.
**FIG. 3C** is a side view of a tube connector, according to an embodiment.
**FIG. 3D** is a side view of a tube connector, according to an embodiment.
**FIG. 3E** is a side view of a tube connector, according to an embodiment.
**FIG. 3F** is a side view of a tube connector, according to an embodiment.
**FIG. 3G** is a side view of a tube connector, according to an embodiment.
**FIG. 4** is a flow diagram of a method for assembling a portable urine collection system, according to an embodiment.

### DETAILED DESCRIPTION

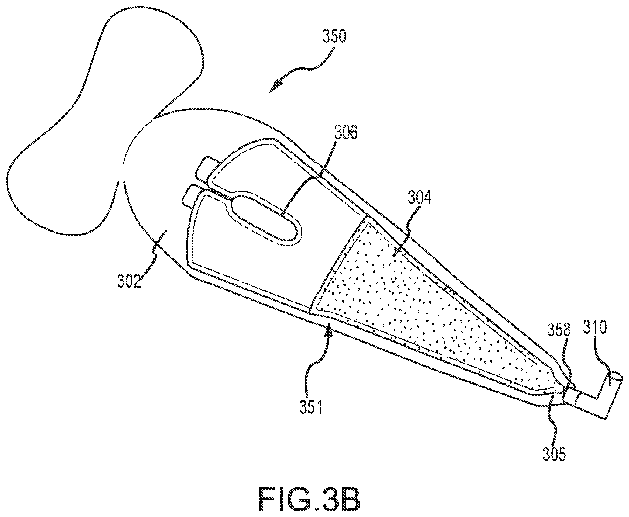

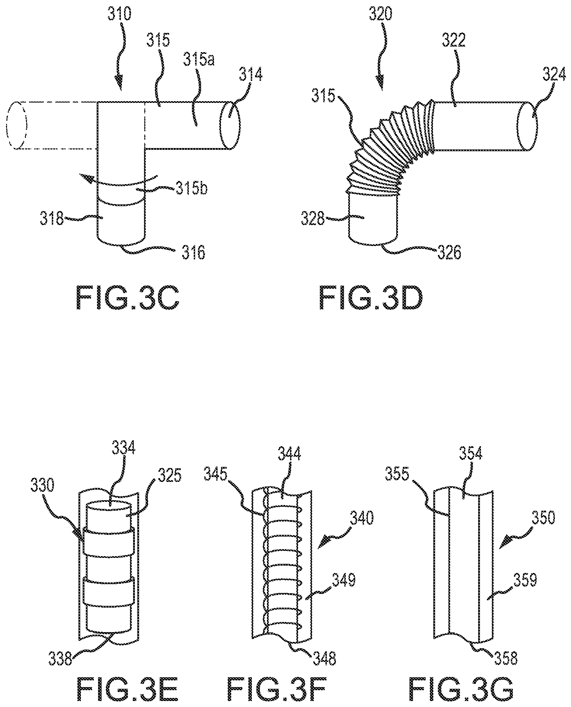

Embodiments disclosed herein are related to pivotable or bendable tube connectors for fluid collection devices, systems, and methods. The embodiments of Figures 3B and 3D-3G show non-claimed systems useful for understanding the invention. Fluid *(e.g.,* urine) collection systems typically include tubing extending between a fluid collection device and a fluid collection or storage container. In conventional systems, when a user is in a supine position with a urine collection device positioned on the pelvic region of a user, the tubing extending between the fluid collection device and the fluid collection or storage container generally angles upwards from the user and the urine collection device. This upwards angling of the tubing may create a bulge under the clothing of the user. Attempting to eliminate the upwards angling of the tubing from the urine collection device may easily kink the tube and/or displace the urine collection device, thus decreasing effectiveness of the urine collection device in collecting and removing urine. At least one, some or all of the embodiments of tube connectors described herein include a feature that provides the technical effect of allowing the one or more tubes providing fluid communication between the urine collection device and the urine storage or collection container to pivot or bend without becoming kinked or pinched. Thus, at least one, some or all of the embodiments of tube connectors described herein result in the technical effect of improving efficiency of the fluid collection systems by inhibiting kinking or pinching of one or more tubes in the fluid collection systems. This pivoting or bending of the tube connectors described herein also result in the technical effect of eliminating the unsightly and/or uncomfortable bulge present in conventional urine collection systems without reducing the efficiency of the urine collection system. In some embodiments, the tube connector includes one or more flexible materials that are easily deformable by the user or caregiver and that retain the shape or position to which the tube connector has been deformed or bent without additional support. Although reference is made in the description and the figures to urine collection devices, the tube connectors described herein also may be used in other fluid collection and removal devices, such as wound care devices that remove fluid from a wound.

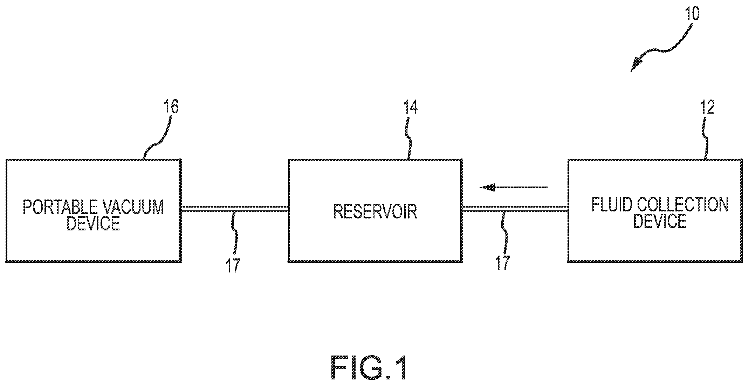

**FIG. 1** is a block diagram of a fluid collection system 10, according to an embodiment. The fluid collection system 10 may be included in any of the embodiments of fluid collection systems described herein. The system 10 includes a fluid (*e.g.*, urine) collection device 12 *(e.g.,* any of the fluid collection assemblies disclosed herein), a urine collection container 14 *(e.g.,* reservoir), and a pump 16 *(e.g.,* portable vacuum device or vacuum). The fluid collection device 10, the urine collection container 14, and the pump 16 may be fluidly coupled to each other via one or more tubes 17 or conduits. For example, fluid collection device 10 may be operably coupled to one or more of the urine collection container 14 or the pump 16 via the tube 17. In some embodiments, the pump 16 may be secured directly to the urine collection container 14. Fluid *(e.g.,* urine or other bodily fluids) collected in the fluid collection device 10 may be removed from the fluid collection device 10 via the tube 17 secured to the fluid collection device 12. Suction force may be introduced into the chamber of the fluid collection device 12 via the inlet of the tube 17 responsive to suction (*e.g*., vacuum) force applied at the outlet of the tube 17.

The suction force may be applied to the outlet of the tube 17 by the pump 16 either directly or indirectly. The suction force may be applied indirectly via the urine collection container 14. For example, the outlet of the tube 17 may be disposed within or fluidly coupled to an interior region of the urine collection container 14 and an additional tube 17 may extend from the urine collection container 14 to the pump 16. Accordingly, the pump 16 may apply suction to the fluid collection device 12 via the urine collection container 14. The suction force may be applied directly via the pump 16. For example, the outlet of the tube 17 may be disposed within the pump 16. An additional tube 17 may extend from the pump 16 to a point outside of the fluid collection device 12, such as to the urine collection container 14. In such examples, the pump 16 may be disposed between the fluid collection device 12 and the urine collection container 14.

The urine collection container 14 is sized and shaped to retain a fluid therein. The urine collection container 14 may include a bag (*e.g.*, drainage bag), a bottle or cup *(e.g.,* collection jar), or any other enclosed container for storing bodily fluid(s) such as urine. In some examples, the tube 17 may extend from the fluid collection device 12 and attach to the urine collection container 14 at a first point therein. An additional tube 17 may attach to the urine collection container 14 at a second point thereon and may extend and attach to the pump 16. Accordingly, a vacuum (*e.g*., suction) may be drawn through fluid collection device 12 via the urine collection container 14. Fluid, such as urine, may be drained from the fluid collection device 12 using the pump 16.

The pump 16 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 16 may provide a vacuum or suction to remove fluid from the fluid collection device 12. In some examples, the pump 16 may be powered by one or more of a power cord *(e.g.,* connected to a power socket), one or more batteries, or even manual power *(e.g.,* a hand operated vacuum pump). In some examples, the pump 16 may be sized and shaped to fit outside of, on, or within the fluid collection device 12. For example, the pump 16 may include one or more miniaturized pumps or one or more micro pumps. The vacuum sources disclosed herein may include one or more of a switch, a button, a plug, a remote, or any other device suitable to activate the pump 16.

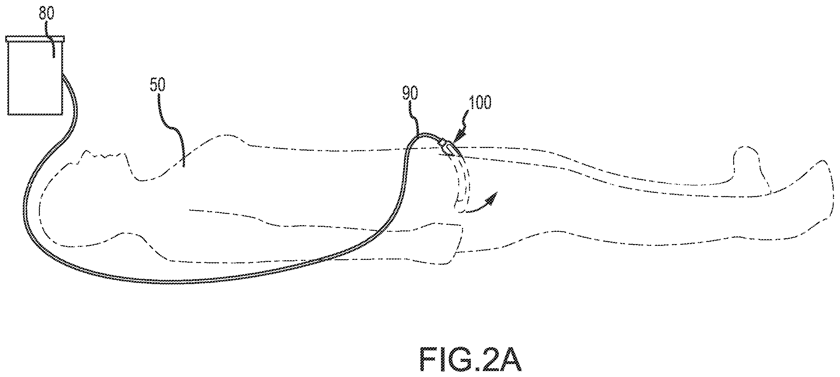

Turning to **FIG. 2A****,** which is a side view of a conventional urine collection system positioned on a user 50 in a supine position. The urine collection system may include a urine collection device 100 positioned on the pelvic region of the user 50 and a tube 90 extending from the urine collection device 100 to the urine storage container 80. The tube 90 generally angles upwards from the user 50 and the urine collection device 100 and creates a bulge under the clothing of the user. Attempting to eliminate the upwards angling of the tube 90 from the urine collection device 100 may easily kink the tube and/or displace the urine collection device, thus decreasing effectiveness of the urine collection device in collecting and removing urine. For example, attempting to flatten the bulge created by the tube 90 may direct a distal end of the urine collection device 100 outward, away from the user 50.

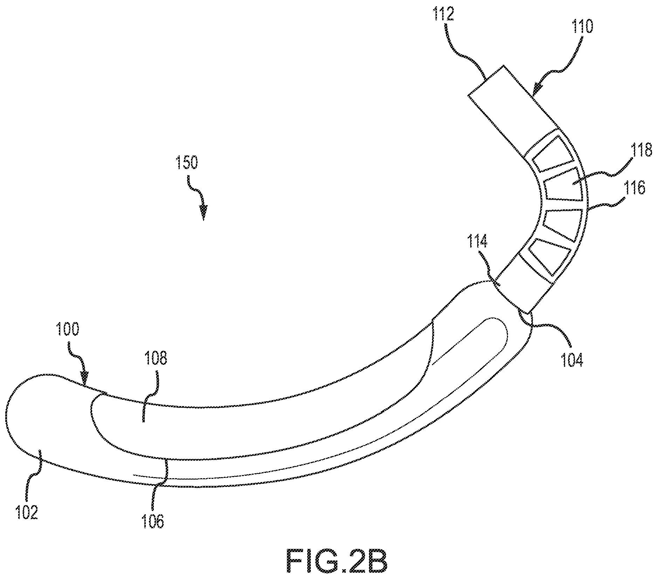

**FIG. 2B** is side view of a urine collection system 150 including a urine collection device 100 having a sump therein and a tube connector 110, according to an embodiment. The urine collection device 100 includes a female urine collection device 100 having a fluid impermeable barrier 102 defining an opening 106. The urine collection device 100 also may include an opening or port 104 sized to receive a portion of the tube connector 110 or the tube 90 (shown in **FIG. 2A****).** The urine collection device 100 of the urine collection system 150 includes a permeable body 108 or support positioned within a chamber defined by the fluid impermeable barrier 102. The opening 106 is positioned such that the permeable layer 108 or body extends across at least a portion *(e.g.,* all) of the opening 106 and the permeable layer 108 may be positioned proximate (*e.g*., adjacent) to the urethra of the user 50. The urine collection device 100 includes a sump within a chamber defined by the fluid impermeable barrier 102. The sump includes an area or region within the chamber where urine discharged through the opening 106 is collected in the urine collection device 100 for removal through the tube. For example, the sump in the urine collection device 100 may include an area within the urine collection device 100 distal to the port 104, such that urine discharged through the opening 106 falls and/or is vacuumed down to the sump for removal.

Although the tube connector 110 is shown secured to the female urine collection device 100 in the system 150, the tube connector 110 may be secured or securable to a male urine collection device, such as the urine collection devices 301, 351 described below. The tube connector 110 may be secured or securable to other male or female urine collection devices or vacuum assisted skin care devices. PCT International Application No. PCT/US2019/029616, U.S. Patent Application No. 15,260,103, U.S. Patent Application No. 16/433,773, and U.S. Provisional Patent Application No. 63/067542 describe various embodiments of both male and female fluid collection devices, including fluid permeable and fluid impermeable materials, that may be used in any of the embodiments disclosed herein. U.S. Patent Application No. 15/384,196 describe various embodiments of skin care devices that may be used in any of the embodiments disclosed herein.

The tube connector 110 may be secured or securable to the urine collection device 100 and may be positioned at least partially outside the urine collection device 100. In some embodiments, the tube connector 110 has a first end region 114 proximate to the urine collection device 100 and a second end region 112 distal to the urine collection device 100. The tube connector 110 is in fluid communication with the sump within the urine collection device 100. In some embodiments, a portion of the tube connector 110 extends through the port 110 into the urine collection device 100 such that the first end region 114 is positioned within the sump or proximate to the sump within the chamber defined by the fluid impermeable barrier 102. In some embodiments, a urine collection device tube may extend from or proximate to the sump within the urine collection device 100 and through the port 104, and the first end region 114 of the tube connector 110 may be secured to the urine collection device tube outside of the urine collection device 100. The urine collection device tube, then, may provide fluid communication between the tube connector 110 and the sump in the urine collection device 100.

The tube connector 110 is adjustable between multiple orientations that adjust positioning of the second end region 112 relative to the first end region 114 without inhibiting (*e.g*., whilst maintaining, whilst functionally maintaining, or whilst meaningfully maintaining) the fluid communication between the second end region 112 and the sump in the urine collection device 100. For example, the tube connector may be configured to allow the second end region 112 about an axis extending through the first end region 114 and/or allow the second end region 112 to be angled at selected angles relative to the first end region 114. In some embodiments, the tube connector 110 includes an articulated region 116 having multiple tubular members 118 secured together such that the tube connector 110 is adjustable between multiple orientations that adjust positioning of the second end region 112 relative to the first end region 114 without inhibiting the fluid communication between the second end region 112 and the sump within the urine collection device 100. Accordingly, the tube connector 110 may be adjustable between a first orientation having the second end region 112 angled a first amount from the first end region 114 of and at least a second orientation having the second end region 112 angled a second amount from the first end region 114 different than the first amount without inhibiting the fluid communication between the second end region 112 and the sump.

Each of the multiple tubular members 118 may be positioned within a conduit defined by the tube connector 110 and/or at least partially embedded in a material of the articulated region 116 in the tube connector 110 shown in **FIG. 2B****.** In some embodiments, each of the multiple tubular members 118 may be spaced from adjacent tubular members 118. In some embodiments, each of the multiple tubular members 118 may contact and/or be pivotably secured to adjacent tubular members 118. As shown in **FIG. 2B****,** at least a portion of the multiple tubular members 118 may include multiple angle cut tubular members. The angle cut tubular members 118 may provide the technical effect of allowing the articulated region 116 to bend such that the first end region 114 and the second end region 112 are generally perpendicular to one another without the tube connector 110 kinking and disrupting fluid communication between the second end region 112 and the sump in the urine collection device. The multiple tubular members 118 of the articulated region 116 may be secured to one another and/or a material of the articulated region 116 resulting in the technical effect of allowing the tube connector 110 to be pivoted or bent to a selected angle at the articulated region 116 and retain at that selected angle until a user or caregiver pivots or bends the articulated region to a different selected angle.

In some embodiments, each of the multiple tubular members 118 may include a generally rigid material, such as plastic or metal. The tube connector 110 is flexible and may include a rubber or plastic material. For example, the tube connector 110 may include a material including one or more of silicone, polyvinyl chloride (PVC), polyethylene, polypropylene plastic, and/or plasticizers. In some embodiments, the multiple tubular members 118 are positioned within a conduit defined by the rubber or plastic material of the articulated region 116 and/or may be at least partially embedded in the rubber or plastic material of the articulated region 116. In some embodiments, the multiple tubular members 118 include the flexible rubber or plastic materials described above, and the multiple tubular members 118 may be connected by a fluid impermeable membrane material in the articulated region 116. In embodiments having the multiple tubular members 118 pivotably secured together, the multiple tubular members 118 may define a conduit extending partially between the first end region 114 and the second end region 112, and the rubber or plastic material may be generally absent from the articulated region 116.

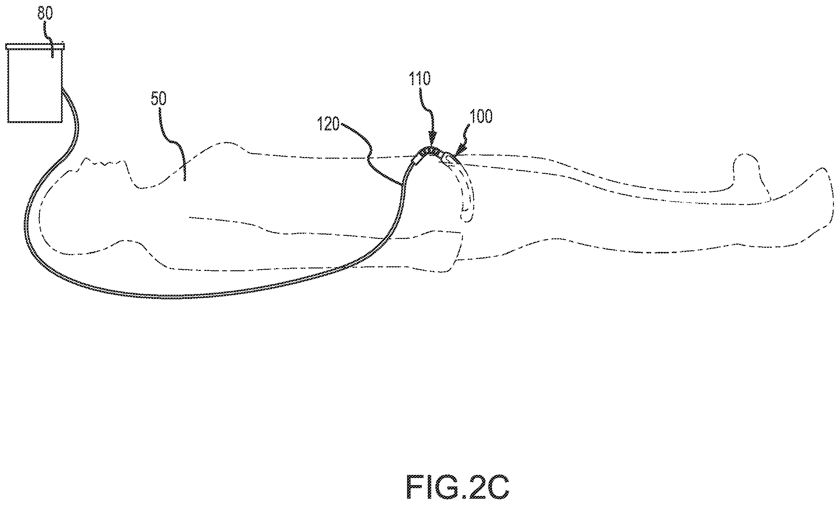

Turning to **FIG. 2C****,** when the tube connector 110 may provide the technical effect of allowing the tube to connector to be bent by pivoting the second end region 112 relative to the first end region 114, thereby eliminating the unsightly and/or uncomfortable bulge from the tube 120 without reducing the efficiency of the urine collection system 150. The tube connector 110 also may provide the technical effect of inhibiting the distal end of the urine collection device 100 from swinging out away from the user 50 without disrupting fluid communication between the tube 120 and the sump in the urine collection device 100. Thus, a vacuum source (not shown), may draw urine from the sump in the urine collection device 100, through the tube connector 110 and the tube 120, and into a urine collection or storage container 80. Although shown with the tube connector 110, the urine collection system may include any of the tube connectors 110, 310, 320, 330, 340 described herein used with the urine collection device 100, wherein the urine collection systems with tube connectors 110, 320, 330, 340 are non-claimed embodiments useful to understanding the invention.

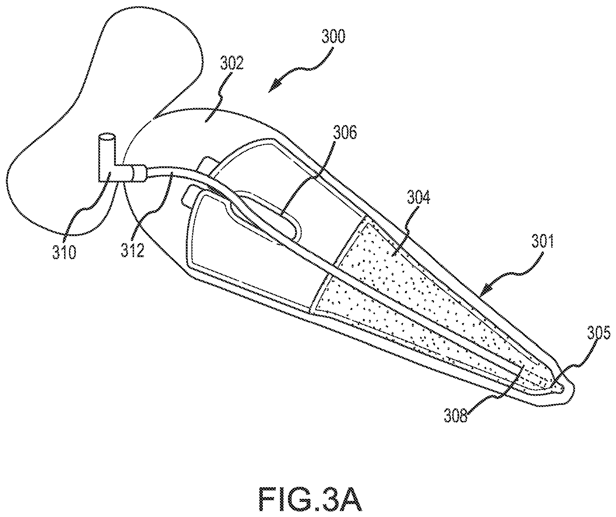

**FIG. 3A** is a front view of a urine collection system 300 including a male urine collection device 301 having a sump 305 therein and a tube connector 310 that is adjustable between multiple orientations without inhibiting the fluid communication between the tube connector 310 and the sump 305. Although shown with the tube connector 310, the urine collection system 300 may include any of the tube connectors 110, 310, 320, 330, 340 described herein, wherein the urine collection systems with tube connectors 110, 320, 330, 340 are non-claimed embodiments useful to understanding the invention. The male urine collection device 301 may include a fluid impermeable barrier 302 defining an opening 306 sized to receive at least a portion of a penis therethrough. The fluid impermeable barrier may define a chamber that narrows from the open 306 to a tip distal to the opening 306. The urine collection device 301 may include a fluid permeable material 304 positioned within the chamber defined by the fluid impermeable barrier 302. The fluid permeable material 304 may extend at least partially between the opening 306 and the sump 305 at the narrow end of the chamber. In some embodiments, the fluid permeable layer 304 or body narrows complementary to the narrowing of the chamber. PCT International Application No. PCT/US2019/029616 describes various materials that may be included in a fluid permeable layer.

The urine collection system 300 also may include a tube 312 extending from the sump 305 in the chamber, through a port 308 in the fluid impermeable barrier 302, and out of the urine collection device 301. In some embodiments, the tube may extend from the sump 305, through the opening 306, and out of the urine collection device 301 *(e.g.,* the port 308 is absent). The tube 312 may include any of the plastic or rubber materials described above in relation to the tube connector 110.

The urine collection system 300 includes a tube connector 310 that provides the technical effect of allowing the tube connector to be adjusted between multiple orientations without inhibiting the fluid communication between the tube connector 310 and the sump 305. **FIG. 3C** shows a side view of the tube connector detached from the tube 312. The tube connector 310 includes a first end region 316 and a second end region 314 that is pivotable relative to the first end region 316. The tube connector 310 includes a base 318 at the first end region 316 that is configured to detachably secure to the tube 312. For example, an end of the tube 312 may be inserted into the base 318 effective to provide fluid communication between the tube connector 310 and the tube 312. In some embodiments, at least a portion of the base 318 may be inserted into an end of the tube 312 effective to provide fluid communication between the tube connector 310 and the tube 312.

The tube connector 310 also includes an elbow pivotably secured to the base 318. The elbow 315 may include a first arm 315a positioned proximate to the base 318 and a second arm 315b at the second end region 314 of the tube connector 310. The first arm 315a and the second arm 315b of the elbow 315 may be angled relative to one another, such as the second arm 315b being substantially perpendicular to the first arm 315a (and the base 318). The elbow 315 is pivotably coupled to the base 318 such that the second arm 315b of the elbow 315 and the second end region 314 may rotate about an axis extending through the base 318 and the first arm 315a of the elbow 315. In some embodiments, the second end region 314 may rotate at least about 90 degrees, at least about 180 degrees, at least about 270 degrees, or about 360 degrees around the axis extending through the first arm 315a and the base 315b. The base 318 may remain substantially stationary as the second end region 314 rotates about the axis.

The base 318 and the elbow 315 may define a conduit extending through the tube connector 310. The second end region 314 may be configured to detachably secure to a vacuum tube (not shown) to provide fluid communication between the tube 312 and the vacuum tube through the tube connector 310. For example, an end of the vacuum tube may be inserted into the second end region 314 effective to provide fluid communication between the tube connector 310, the sump 305, and the vacuum tube. In some embodiments, at least a portion of the second end region 314 may be inserted into an end of the vacuum tube effective to provide fluid communication between the tube connector 310 and the vacuum tube.

Turning to **FIG. 3B****,** a urine collection system 350 may include a urine collection device 351 and the tube connector 310. The urine collection device 351 may include any aspect of the urine collection device 301 described above, such as the fluid impermeable barrier 302, the opening 306, and the fluid permeable layer 304. The urine collection device 351 may include a port 358 positioned at or proximate to the narrow end of the chamber defined by the fluid impermeable barrier 302, and the port 308 of the urine collection device 301 may be absent. In some embodiments, the port 358 is space from the narrow end or tip of the chamber.

The tube connector 310 may be positioned proximate to the port 358 in the urine collection system 350. In some embodiments, the base 318 of the tube connector may extend through the port 358 into the chamber to provide fluid communication between the tube connector 310 and the sump 305 in the urine collection device 350. The tube connector 310 may be fixedly secured to the urine collection device 351, such as fixedly secured to the fluid impermeable barrier 302 proximate to the port 358. In some embodiments, a urine collection device tube may be secured to the base 318 of the tube connector 310 and may extend through the port 358 and into the chamber to provide fluid communication between the sump 305 in the urine collection device 351 and the tube connector 310. Although shown with a single tube connector 310, urine collection systems may include multiple tube connectors 310 in a series.

**FIG. 3D** is a side view of a tube connector 320, according to a non-claimed embodiment useful for understanding the invention. Unless otherwise noted, the tube connector 320 may include any aspect of other tube connectors described herein. In some embodiments, the tube connector 320 includes a first end region 326 and a second end region 324 distal to and/or spaced from the first end region 326. The tube connector 320 may be securable or fixedly secured to a urine collection device 100, 301, 351 or other urine collection devices. The tube connector 320 may be positioned at least partially outside the urine collection device with the first end region 326 proximate to the urine collection device and the second end region 324 distal to the urine collection device.

When secured to the urine collection device, the tube connector 320 is adjustable between multiple orientations that result in the technical effect of allowing adjustment of the second end region 324 relative to the first end region 326 without inhibiting the fluid communication between the second end region 322 and the sump 305 within the urine collection device. In some embodiments, the tube connector 320 includes a base 328 at the first end region 326, a head 322 at the second end region 322, and a bellows segment 315 positioned between the base 328 and the head 322. The bellows segment 315 allows the tube connector 320 to be adjustable between multiple orientations that adjust positioning of the second end region 324 relative to the first end region 326 without inhibiting the fluid communication between the second end region 314 and the sump 305. **FIG. 3D** shows the head 322 and the base 328 substantially perpendicular to one another. The bellows segment 315 allows the tube connector 310 to bend to selected orientations with the head 322 moved and/or angled relative to the base 328. For example, the bellows segment 315 may bend to allow the head 322 to be pivoted and angled anywhere between 1 and 360 degrees relative to the base 328. Once bent to a selected orientation, the second end region 322 may remain substantially in that orientation relative to the first end region 326 until a user or caregiver further adjusts the tube connector 320.

In some embodiments, the base 328 at the first end region 326 may be configured to detachably secure to the urine collection device tube (not shown) positioned at least partially in the urine collection device and extending out of the urine collection device. For example, an end of the urine collection device tube may be inserted into the base 328 effective to provide fluid communication between the tube connector 320 and the urine collection device tube. In some embodiments, at least a portion of the base 328 may be inserted into an end of the urine collection device tube effective to provide fluid communication between the tube connector 320 and the urine collection device tube.

The head 322 at the second end region 324 may be configured to detachably secure to a vacuum tube (not shown) to provide fluid communication between the sump 305 and the vacuum tube through the tube connector 320. For example, an end of the vacuum tube may be inserted into the second end region 324 effective to provide fluid communication between the tube connector 320, the sump, and the vacuum tube. In some embodiments, at least a portion of the head 322 may be inserted into an end of the vacuum tube effective to provide fluid communication between the tube connector 320 and the vacuum tube.

**FIG. 3E** is a side view of a portion of a tube connector 330, according to a non-claimed embodiment useful for understanding the invention. The portion of the tube connector 330 shown in **FIG. 3E** may replace the bellows segment 315 of the tube connector 320 and/or the articulated region 116 of the tube connector 110. Unless otherwise noted, the tube connector 330 may include any aspect of the tube connector 110, 310, 320, 340. The tube connector 330 may include a first end region 338 and a second end region 334. The tube connector 330 is adjustable between multiple orientations that result in the technical effect of allowing the tube connector 330 to adjust positioning of the second end region 334 relative to the first end region 338 without inhibiting the fluid communication between the second end region 334 and the sump 305 in the urine collection device. In some embodiments, the tube connector 330 includes an articulated region having multiple tubular members 325 secured together such that the tube connector 330 is adjustable between multiple orientations that adjust positioning of the second end region 334 relative to the first end region 338 without inhibiting the fluid communication between the second end region 334 and the sump 305 within the urine collection device. Each of the multiple tubular members 325 may be positioned within a conduit defined by the tube connector and/or at least partially embedded in a material of the tube connector 330.

In some embodiments, each of the multiple tubular members 325 may contact and/or be pivotably secured to adjacent tubular members 325. The multiple tubular members 325 of the tube connector 330 may be secured to one another and/or the a material of the tube connector 330 such that the tube connector 330 may be pivoted or bent to a selected angle retain at that selected angle until a user or caregiver pivots or bends the tube connector 330 to a different selected angle.

In some embodiments, each of the multiple tubular members 325 may include a generally rigid material, such as plastic or metal. The tube connector 330 is flexible and may include a rubber or plastic material. For example, the tube connector 330 may include a material including one or more of silicone, polyvinyl chloride (PVC), polyethylene, polypropylene plastic, and/or plasticizers. In some embodiments, the multiple tubular members 325 include the flexible rubber or plastic materials described above, and the multiple tubular members 325 may be connected by a fluid impermeable membrane material in the tube connector 320. In some embodiments, the multiple tubular members 325 are positioned within a conduit defined by the rubber or plastic material of the tube connector 330 and/or may be at least partially embedded in the rubber or plastic material of the tube connector. In embodiments having the multiple tubular members 325 pivotably secured together, the multiple tubular members 325 may define a conduit extending partially between the first end region 338 and the second end region 334, and the rubber or plastic material may be generally absent from the articulated region of the tube connector 330.

**FIG. 3F** is a side view of a portion of a tube connector 340, according to a non-claimed embodiment useful to understanding the invention. The portion of the tube connector 340 shown in **FIG. 3F** may replace the bellows segment 315 of the tube connector 320 and/or the articulated region 116 of the tube connector 110. Unless otherwise noted, the tube connector 340 may include any aspect of the tube connector 110, 310, 320, 330. The tube connector 340 may include a first end region 348 and a second end region 344. The tube connector 340 is adjustable between multiple orientations that results in the technical effect of allowing positional adjustment of the second end region 344 relative to the first end region 348 without inhibiting the fluid communication between the second end region 344 and the sump 305 in the urine collection device.

In some embodiments, the tube connector 340 includes a fluid impermeable and generally flexible tubular member 349 defining a conduit and a spring 345 positioned within the conduit or at least partially embedded within the tubular member 349. The spring 345 may include flexible materials such as metal, plastic, rubber, polyester, polyvinyl chloride, cloth, or combinations thereof. In some embodiments, the spring 345 is absent and the tube connector 340 includes the tubular member 349 having an impermeable membrane covering spun plastic. Spun plastic inside a flexible membrane or material may provide the tube connector 340 with greater flexibility both horizontally and vertically. Moreover, spun plastic inside a flexible membrane or material may provide a soft enough tube connector 340 that a patient will not be injured by inadvertently lying on top of the tube connector 340. Changing the direction of the tube connector 340 also does not displace the fluid collection device to which the tube connector is attached.

**FIG. 3G** is a side view of a portion of a tube connector 350, according to a non-claimed embodiment useful to understanding the invention. The portion of the tube connector 350 shown in **FIG. 3G** may replace the bellows segment 315 of the tube connector 320 and/or the articulated region 116 of the tube connector 110. Unless otherwise noted, the tube connector 350 may include any aspect of the tube connectors 110, 310, 320, 330, 340. The tube connector 350 may include a first end region 358 and a second end region 354. The tube connector 350 is adjustable between multiple orientations that result in the technical effect of allowing positional adjustment of the second end region 354 relative to the first end region 358 without inhibiting the fluid communication between the second end region 354 and the sump 305 in the urine collection device.

In some embodiments, the tube connector 350 includes the tubular member 349 having an impermeable membrane covering spun plastic 355. The spun plastic 355 inside a flexible membrane or material may provide the tube connector 350 with greater flexibility both horizontally and vertically. Moreover, the spun plastic 355 inside a flexible membrane or material may provide a soft enough tube connector 350 that a patient will not be injured by inadvertently lying on top of the tube connector 350. Changing the direction of the tube connector 350 also does not displace the fluid collection device to which the tube connector is attached.

In some embodiments, the generally flexible tubular member 359 of the tube connector 350 includes one or more flexible materials that are easily deformable by the user or caregiver and that retain the shape or position to which the tube connector 350 has been deformed or bent without additional support. In some embodiments, the spun plastic 355 may be absent. The tube connector 350 may include one or more support materials such as metal, plastic, rubber, polyester, polyvinyl chloride, cloth, or combinations thereof. The one or more support materials may be inserted into the tube connector 340, such as extruded in linear lines parallel to an axis through the tube connector 350 and/or perpendicular to the axis of the tube connector 350. In some embodiments, one or more portions of the tubular member 359 include a thinner or thicker wall thickness relative to a wall thickness of the tube 90 and/or other portions of the tubular member 359 that allows the tube connector 350 to be easily deformable by the user or caregiver and also retain the shape or position to which the tube connector 350 has been deformed.

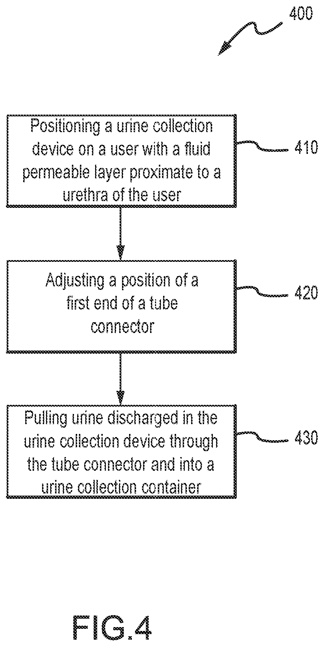

**FIG. 4** is a flow diagram of a method 400 for assembling a portable urine collection system, according to an embodiment. The method 400 includes an act 410 of positioning a urine collection device on a user with a fluid permeable body of the urine collection device proximate to a urethra of the user, the urine collection device including a sump positioned within the urine collection device and a fluid impermeable barrier. The method 400 also includes an act 420 of adjusting a position of a first end region of a tube connector in fluid communication with the sump relative to a second end region of the tube connector without inhibiting the fluid communication between the second end region and the sump to orient the tube connector to a selected orientation. The method also includes an act 430 of pulling urine discharged in the urine collection device from the sump, through the tube connector, and into a urine collection container.

In some embodiments, the act 410 of positioning a urine collection device on a user with a fluid permeable body of the urine collection device at least proximate to a urethra of the user includes positioning the urine collection device on a female user with the fluid permeable body of the urine collection device proximate to the urethra of the user through an opening defined by the fluid impermeable barrier. In some embodiments, the act 410 of positioning a urine collection device on a user with a fluid permeable body of the urine collection device proximate to a urethra of the user includes inserting a penis of a male user through an opening in the fluid impermeable barrier to position the urethra of the male user proximate to the fluid permeable body.

In some embodiments, the act 420 of adjusting a position of a first end region of a tube connector in fluid communication with the sump relative to a second end region of the tube connector includes rotating an elbow of the tube connector relative to a base of the tube connector.

The method 400 includes an act of detachably securing a urine collection device tube to the first end region of the tube connector, the urine collection device tube extending into the sump and providing fluid communication between the sump and the tube connector. In some embodiments, the method 400 includes an act of securing a vacuum tube to the second end region of the tube connector to provide fluid communication between the tube connector and a vacuum source.

The acts of the method 400 described above are for illustrative purposes. For example, the acts of the method 400 can be performed in different orders, split into multiple acts, modified, supplemented, or combined. In an embodiment, one or more of the acts of the method 400 can be omitted from the method 400. Any of the acts of the method 400 can include using any of the urine collection systems disclosed herein. Only methods falling under the scope of the claims are methods according to the invention.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated as long as they fall under the scope of the claims. The various aspects and embodiments disclosed herein are for purposes of illustration and are not intended to be limiting.

## Claims

1. A fluid collection system (10, 150, 300), comprising:
a fluid collection device (100, 301) including a sump (305) positioned within the fluid collection device, a fluid impermeable barrier (102, 302) at least partially defining an opening (106, 306), and a fluid permeable body (108, 304) within the fluid collection device positioned to be at least proximate to skin of a user when the fluid collection device is in use; and
a tube connector (310) positioned at least partially outside the fluid collection device and in fluid communication with the sump, the tube connector having a first end region (316) proximate to the fluid collection device and a second end region (314) distal to the fluid collection device that is in fluid communication with the sump (305),
wherein the tube connector (310) includes a base (318) including the first end region (316) and an elbow (315) including the second end region (314), the elbow (315) being pivotably secured to the base (318), the tube connector being adjustable between multiple orientations that adjust positioning of the second end region relative to the first end region whilst functionally maintaining the fluid communication between the second end region and the sump (305); and
further comprising a fluid collection device tube (312) detachably secured to the first end region (316) of the tube connector (310), the fluid collection device tube extending into the sump (305) and providing fluid communication between the sump (305) and the tube connector (310).

2. The fluid collection system of claim 1, further comprising a vacuum tube secured or securable to the second end region of the tube connector and configured to provide fluid communication between the tube connector and a vacuum source (16).

3. The fluid collection system of claim 2, further comprising a fluid collection container (14) and the vacuum source (16) configured to draw fluid discharged in the fluid collection device out of the fluid collection device, through the tube connector, and through the vacuum tube into the fluid collection container.

4. The fluid collection system of any of claims 1-3, wherein the fluid collection device is configured as a female external catheter urine collection device (100) and the fluid permeable body (108) is positioned within the female external catheter urine collection device (100) to be disposed at least proximate to a urethra of a female user.

5. The fluid collection system of any of claims 1-3, wherein the fluid collection device is configured as a male external urine collection device (301) and the fluid permeable body (304) is positioned within the male external catheter urine collection device to be disposed at least proximate to a urethra of a male user.

6. The fluid collection system of claim 5, wherein the sump and the tube connector are positioned at a distal end region of the male external urine collection device (301).

7. The fluid collection system of claim 6, further comprising an additional tube connector secured or securable to a proximate end region of the male external urine collection device (301) and an additional tube (17) providing fluid communication between the tube connector and the additional tube connector.

8. The fluid collection system of any of claims 1-3, wherein the fluid collection device is configured as a fluid collection device for removing wound drainage.

9. The fluid collection system of any of claims 1-8, wherein the tube connector (310) being adjustable between multiple orientations that adjust positioning of the second end region relative to the first end region whilst functionally maintaining the fluid communication between the second end region and the sump (305) includes at least one of:
the second end region (314) being rotatable about an axis extending through the first end region (316) such that the connector is adjustable between a first orientation having the second end region of the tube connector (310) at a first position and a second orientation having the second end region of the tube connector at a second position rotated at least about 90 degrees from the first position whilst functionally maintaining the fluid communication between the second end region and the sump.

10. A method of collecting fluid, comprising:
positioning a fluid collection device (100, 301) on a user with a fluid permeable body (108, 304) of the fluid collection device proximate to skin of the user, the fluid collection device including a sump positioned within the fluid collection device and a fluid impermeable barrier (102,302); the fluid collection device further comprising a fluid collection device tube detachably secured to the first end region of a tube connector (310), the fluid collection device tube extending into the sump and providing fluid communication between the sump and the tube connector;
adjusting a position of a first end region (316) of the tube connector (310) in fluid communication with the sump relative to a second end region (314) of the tube connector (310) whilst functionally maintaining the fluid communication between the second end region and the sump to orient the tube connector to a selected orientation, wherein the tube connector includes a base (318) including the first end region and an elbow (315) including the second end region, the elbow being pivotably secured to the base; and
drawing fluid discharged in the fluid collection device from the sump (305), through the tube connector (310), and into a fluid collection container (14).

## Patentansprüche

1. Flüssigkeitssammelsystem (10, 150, 300), umfassend:
eine Flüssigkeitssammelvorrichtung (100, 301), die einen innerhalb der Flüssigkeitssammelvorrichtung positionierten Sumpf (305), eine flüssigkeitsundurchlässige Barriere (102, 302), die mindestens teilweise eine Öffnung (106, 306) definiert, und einen flüssigkeitsdurchlässigen Körper (108, 304) innerhalb der Flüssigkeitssammelvorrichtung, der so positioniert ist, dass er sich mindestens in der Nähe der Haut eines Benutzers befindet, wenn die Flüssigkeitssammelvorrichtung in Verwendung ist, einschließt; und
einen Schlauchverbinder (310), der mindestens teilweise außerhalb der Flüssigkeitssammelvorrichtung positioniert ist und in fließender Verbindung mit dem Sumpf steht, wobei der Schlauchverbinder einen ersten Endbereich (316) in der Nähe der Flüssigkeitssammelvorrichtung und einen zweiten Endbereich (314) distal der Flüssigkeitssammelvorrichtung, der in fließender Verbindung mit dem Sumpf (305) steht, aufweist,
wobei der Schlauchverbinder (310) eine Basis (318), die den ersten Endbereich (316) einschließt, und ein Winkelstück (315), das den zweiten Endbereich (314) einschließt, einschließt, wobei das Winkelstück (315) schwenkbar an der Basis (318) gesichert ist, wobei der Schlauchverbinder zwischen mehreren Ausrichtungen einstellbar ist, die eine Positionierung des zweiten Endbereichs relativ zum ersten Endbereich einstellen, während die fließende Verbindung zwischen dem zweiten Endbereich und dem Sumpf (305) funktionsfähig aufrechterhalten wird; und
weiter umfassend einen Flüssigkeitssammelvorrichtungsschlauch (312), der lösbar am ersten Endbereich (316) des Schlauchverbinders (310) gesichert ist, wobei sich der Flüssigkeitssammelvorrichtungsschlauch in den Sumpf (305) erstreckt und eine fließende Verbindung zwischen dem Sumpf (305) und dem Schlauchverbinder (310) bereitstellt.

2. Flüssigkeitssammelsystem nach Anspruch 1, weiter umfassend einen Vakuumschlauch, der am zweiten Endbereich des Schlauchverbinders gesichert oder sicherbar ist und dazu ausgebildet ist, eine fließende Verbindung zwischen dem Schlauchverbinder und einer Vakuumquelle (16) bereitzustellen.

3. Flüssigkeitssammelsystem nach Anspruch 2, weiter umfassend einen Flüssigkeitssammelbehälter (14) und die Vakuumquelle (16), die dazu ausgebildet ist, die in die Flüssigkeitssammelvorrichtung abgelassene Flüssigkeit aus der Flüssigkeitssammelvorrichtung, durch den Schlauchverbinder und durch den Vakuumschlauch in den Flüssigkeitssammelbehälter zu saugen.

4. Flüssigkeitssammelsystem nach einem der Ansprüche 1-3, wobei die Flüssigkeitssammelvorrichtung als eine Urinsammelvorrichtung (100) für einen externen Katheter für Frauen ausgebildet ist und der flüssigkeitsdurchlässige Körper (108) innerhalb der Urinsammelvorrichtung (100) für einen externen Katheter für Frauen so positioniert ist, dass er mindestens in der Nähe der Harnröhre eines weiblichen Benutzers angeordnet ist.

5. Flüssigkeitssammelsystem nach einem der Ansprüche 1-3, wobei die Flüssigkeitssammelvorrichtung als eine externe Urinsammelvorrichtung (301) für Männer ausgebildet ist und der flüssigkeitsdurchlässige Körper (304) innerhalb der Urinsammelvorrichtung für einen externen Katheter für Männer so positioniert ist, dass er mindestens in der Nähe der Harnröhre eines männlichen Benutzers angeordnet ist.

6. Flüssigkeitssammelsystem nach Anspruch 5, wobei der Sumpf und der Schlauchverbinder an einem distalen Endbereich der externen Urinsammelvorrichtung (301) für Männer positioniert sind.

7. Flüssigkeitssammelsystem nach Anspruch 6, weiter umfassend einen zusätzlichen Schlauchverbinder, der an einem nahegelegenen Endbereich der externen Urinsammelvorrichtung (301) für Männer gesichert oder sicherbar ist, und einen zusätzlichen Schlauch (17), der eine fließende Verbindung zwischen dem Schlauchverbinder und dem zusätzlichen Schlauchverbinder bereitstellt.

8. Flüssigkeitssammelsystem nach einem der Ansprüche 1-3, wobei die Flüssigkeitssammelvorrichtung als eine Flüssigkeitssammelvorrichtung zum Entfernen von Wunddrainage ausgebildet ist.

9. Flüssigkeitssammelsystem nach einem der Ansprüche 1-8, wobei der Schlauchverbinder (310), der zwischen mehreren Ausrichtungen einstellbar ist, die eine Positionierung des zweiten Endbereichs relativ zum ersten Endbereich einstellen, während die fließende Verbindung zwischen dem zweiten Endbereich und dem Sumpf (305) funktionsfähig aufrechterhalten wird, mindestens eines einschließt von:
dem zweiten Endbereich (314), der um eine sich durch den ersten Endbereich (316) erstreckende Achse drehbar ist, sodass der Verbinder zwischen einer ersten Ausrichtung, die den zweiten Endbereich des Schlauchverbinders (310) in einer ersten Position aufweist, und einer zweiten Ausrichtung, die den zweiten Endbereich des Schlauchverbinders in einer zweiten Position aufweist, die gegenüber der ersten Position um mindestens etwa 90 Grad gedreht ist, einstellbar ist, während die fließende Verbindung zwischen dem zweiten Endbereich und dem Sumpf funktionsfähig aufrechterhalten wird.

10. Verfahren zum Sammeln von Flüssigkeit, umfassend:
Positionieren einer Flüssigkeitssammelvorrichtung (100, 301) auf einem Benutzer, wobei ein flüssigkeitsdurchlässiger Körper (108, 304) der Flüssigkeitssammelvorrichtung in der Nähe der Haut des Benutzers ist, wobei die Flüssigkeitssammelvorrichtung einen innerhalb der Flüssigkeitssammelvorrichtung positionierten Sumpf und eine flüssigkeitsundurchlässige Barriere (102, 302) einschließt; wobei die Flüssigkeitssammelvorrichtung weiter einen Flüssigkeitssammelvorrichtungsschlauch umfasst, der lösbar am ersten Endbereich des Schlauchverbinders (310) gesichert ist, wobei sich der Flüssigkeitssammelvorrichtungsschlauch in den Sumpf erstreckt und eine fließende Verbindung zwischen dem Sumpf und dem Schlauchverbinder bereitstellt;
Einstellen einer Position eines ersten Endbereichs (316) des Schlauchverbinders (310), der in fließender Verbindung mit dem Sumpf steht, relativ zu einem zweiten Endbereich (314) des Schlauchverbinders (310), während die fließende Verbindung zwischen dem zweiten Endbereich und dem Sumpf funktionsfähig aufrechterhalten wird, um den Schlauchverbinder in einer ausgewählten Ausrichtung auszurichten, wobei der Schlauchverbinder eine Basis (318), die den ersten Endbereich einschließt, und ein Winkelstück (315), das den zweiten Endbereich einschließt, einschließt, wobei das Winkelstück schwenkbar an der Basis gesichert ist; und
Saugen der in die Flüssigkeitssammelvorrichtung abgelassenen Flüssigkeit aus dem Sumpf (305), durch den Schlauchverbinder (310) und in einen Flüssigkeitssammelbehälter (14).

## Revendications

1. Système (10, 150, 300) de collecte de fluide, comprenant :
un dispositif (100, 301) de collecte de fluide incluant un puisard (305) positionné au sein du dispositif de collecte de fluide, une barrière (102, 302) imperméable au fluide définissant au moins partiellement une ouverture (106, 306), et un corps (108, 304) perméable au fluide à l'intérieur du dispositif de collecte de fluide positionné pour être au moins proche de la peau d'un utilisateur lorsque le dispositif de collecte de fluide est utilisé ; et
un raccord (310) de tube positionné au moins partiellement à l'extérieur du dispositif de collecte de fluide et en communication fluidique avec le puisard, le raccord de tube présentant une première région d'extrémité (316) à proximité du dispositif de collecte de fluide et une deuxième région d'extrémité (314) distale par rapport au dispositif de collecte de fluide qui est en communication fluidique avec le puisard (305),
dans lequel le raccord (310) de tube inclut une base (318) incluant la première région d'extrémité (316) et un coude (315) incluant la deuxième région d'extrémité (314), le coude (315) étant fixé de manière pivotante à la base (318),
le raccord de tube étant réglable entre plusieurs orientations qui ajustent le positionnement de la deuxième région d'extrémité par rapport à la première région d'extrémité tout en maintenant fonctionnellement la communication fluidique entre la deuxième région d'extrémité et le puisard (305) ; et
comprenant en outre un tube (312) de dispositif de collecte de fluide fixé de manière détachable à la première région d'extrémité (316) du raccord (310) de tube, le tube de dispositif de collecte de fluide s'étendant dans le puisard (305) et fournissant une communication fluidique entre le puisard (305) et le raccord (310) de tube.

2. Système de collecte de fluide selon la revendication 1, comprenant en outre un tube à vide fixé ou pouvant être fixé à la deuxième région d'extrémité du raccord de tube et configuré pour fournir une communication fluidique entre le raccord de tube et une source de vide (16).

3. Système de collecte de fluide selon la revendication 2, comprenant en outre un récipient (14) de collecte de fluide et la source de vide (16) configurée pour aspirer le fluide évacué dans le dispositif de collecte de fluide hors du dispositif de collecte de fluide, à travers le raccord de tube, et à travers le tube de vide dans le récipient de collecte de fluide.

4. Système de collecte de fluide selon l'une quelconque des revendications 1 à 3, dans lequel le dispositif de collecte de fluide est configuré comme un dispositif de collecte d'urine (100) de cathéter externe féminin et le corps (108) perméable au fluide est positionné à l'intérieur du dispositif de collecte d'urine (100) de cathéter externe féminin pour être disposé au moins à proximité d'un urètre d'une utilisatrice féminine.

5. Système de collecte de fluide selon l'une quelconque des revendications 1 à 3, dans lequel le dispositif de collecte de fluide est configuré comme un dispositif de collecte d'urine externe masculin (301) et le corps (304) perméable au fluide est positionné à l'intérieur du dispositif de collecte d'urine de cathéter externe masculin pour être disposé au moins à proximité d'un urètre d'un utilisateur masculin.

6. Système de collecte de fluide selon la revendication 5, dans lequel le puisard et le raccord de tube sont positionnés au niveau d'une région d'extrémité distale du dispositif de collecte d'urine externe masculin (301).

7. Système de collecte de fluide selon la revendication 6, comprenant en outre un raccord de tube supplémentaire fixé ou pouvant être fixé à une région d'extrémité proximale du dispositif de collecte d'urine externe masculin (301) et un tube supplémentaire (17) fournissant une communication fluidique entre le raccord de tube et le raccord de tube supplémentaire.

8. Système de collecte de fluide selon l'une quelconque des revendications 1 à 3, dans lequel le dispositif de collecte de fluide est configuré comme un dispositif de collecte de fluide pour retirer le drainage de plaie.

9. Système de collecte de fluide selon l'une quelconque des revendications 1 à 8, dans lequel le raccord (310) de tube est réglable entre plusieurs orientations qui ajustent le positionnement de la deuxième région d'extrémité par rapport à la première région d'extrémité tout en maintenant fonctionnellement la communication fluidique entre la deuxième région d'extrémité et le puisard (305) inclut au moins l'un parmi :
la deuxième région d'extrémité (314) étant rotative autour d'un axe s'étendant à travers la première région d'extrémité (316) de sorte que le raccord soit réglable entre une première orientation présentant la deuxième région d'extrémité du raccord (310) de tube dans une première position et une deuxième orientation présentant la deuxième région d'extrémité du raccord de tube dans une deuxième position tournée d'au moins environ 90 degrés par rapport à la première position tout en maintenant fonctionnellement la communication fluidique entre la deuxième région d'extrémité et le puisard.

10. Procédé de collecte de fluide, comprenant :
le positionnement d'un dispositif (100, 301) de collecte de fluide sur un utilisateur avec un corps (108, 304) perméable au fluide du dispositif de collecte de fluide à proximité de la peau de l'utilisateur, le dispositif de collecte de fluide incluant un puisard positionné à l'intérieur du dispositif de collecte de fluide et une barrière (102, 302) imperméable au fluide ; le dispositif de collecte de fluide comprenant en outre un tube de dispositif de collecte de fluide fixé de manière détachable à la première région d'extrémité d'un raccord (310) de tube, le tube de dispositif de collecte de fluide s'étendant dans le puisard et fournissant une communication fluidique entre le puisard et le raccord de tube ;
l'ajustement d'une position d'une première région d'extrémité (316) du raccord (310) de tube en communication fluidique avec le puisard par rapport à une deuxième région d'extrémité (314) du raccord (310) de tube tout en maintenant fonctionnellement la communication fluidique entre
la deuxième région d'extrémité et le puisard pour orienter le raccord de tube selon une orientation sélectionnée, dans lequel le raccord de tube inclut une base (318) incluant la première région d'extrémité et un coude (315) incluant la deuxième région d'extrémité, le coude étant fixé de manière pivotante à la base ; et
l'aspiration du fluide évacué dans le dispositif de collecte de fluide à partir du puisard (305), à travers le raccord (310) de tube, et dans un récipient (14) de collecte de fluide.
